# EUROPÄISCHE PATENTANMELDUNG

(11) **EP 0 526 721 A1**
(43) Veröffentlichungstag der Anmeldung: **10.02.1993**
(21) Anmeldenummer: 92110439.4
(22) Anmeldetag: 20.06.1992
(51) Int. Cl.: A61B 1/32

(54) **Endoskop zum Einführen in ein Hohlorgan eines Lebewesens**

(30) Priorität: 03.08.1991 DE 4125806
(71) Anmelder: Richard Wolf GmbH, D-75438 Knittlingen (DE)
(72) Erfinder: Heckele, Helmut, W-7134 Knittlingen (DE); Heimberger, Rudolf, W-7519 Oberderdingen (DE)
(74) Vertreter: Vollmann, Heiko, Dipl.-Ing.

(57) **Zusammenfassung**

Das Endoskop zum Einführen in ein Hohlorgan (10) eines Lebewesens ermöglicht es, das Hohlorgan (10) gegen eine festere äußere Struktur zu ziehen und daran während der Dauer der Behandlung festzulegen. Dazu ist das Endoskop mit einer in dessen Außenschaft (2) einsetzbaren und in diesem geführten Haltevorrichtung mit einer Vielzahl von Hakenelementen (6), an deren distalen Enden sich durch Ausschieben aus dem distalen Ende des Außenschaftes (2) durch Spreizen Haken ausbilden, und mit einer auf dem Außenschaft (2) verstellbaren Anschlagscheibe (3) versehen. Die Spreizung der Hakenelementenden (6) ist optisch kontrollierbar. Zur Fixierung des Hohlorgans (10) wird das Endoskop nach Spreizung der Hakenelementenden (6) bis zur Anlage derselben am Hohlorgan (10) zurückgezogen und in dieser Stellung mittels der Anschlagscheibe (3) festgelegt. Zum Vermessen des Hohlorgans (10) ist eine Meßskala vorgesehen, die eine direkte Ablesung der Spreizstellung der Hakenelementenden im Augenblick des Kontaktes mit der Gewebewand des Hohlorgans ermöglicht.

## Beschreibung

Die Erfindung geht aus Von einem Endoskop zum Einführen in ein Hohlorgan eines Lebewesens, umfassend einen hohlen Außenschaft und eine in diesem angeordnete Optik.

Die Endoskopie des Inneren von Hohlorganen von Menschen und Tieren wird häufig dadurch erschwert, daß die Hohlorgane aufgrund ihrer hohen Flexibilität leicht dem Beobachtungsinstrument ausweichen. Wegen dieser hohen Flexibilität ist oft kein genügender Abstand zwischen Endoskopoptik und dem Zu untersuchenden Bereich des Hohlorgans erreichbar, so daß kein scharfes Bild erzielt werden kann. Auch ist dadurch kaum Raum für die Handhabung von Instrumenten vorhanden. Des weiteren ist es z. B. bei pathologischen Veränderungen von Hohlorganen oft nötig, den noch verbliebenen Freiraum, z. B. bei röhrenförmigen Organen den freien Durchgang, zu vermessen, um entsprechende Behandlungsmaßnahmen vornehmen zu können.

Aus der DE-PS 23 18 860 ist ein Hysteroskop bekannt, welches ausgebildet ist, den Uterus mit Gas aufzudehnen und dann in diesem Zustand mit Hilfe mechanisch wirkender Spreizelemente diesen Zustand aufrechtzuerhalten und das Gas entweichen lassen zu können. Dieses Hysteroskop besteht im wesentlichen aus einem der Abdichtung des Uterus dienenden Adapter, einem Instrumentenschaft, durch den eine Optik einführbar ist und der einen Gasanschluß aufweist. Zum Gespreizthalten sind am distalen Endoskopende Spreizelemente vorgesehen, die gegen Wandbereiche des Uterus zur Anlage bringbar sind. Dabei bestimmen die Anordnung und Form der Spreizelemente das Maß, um den der Uterus freigehalten werden kann. Eine gewisse Stabilisierung des Gewebes ist hiermit zwar erreichbar, doch keine Fixierung des Hohlorgans im eigentlichen Sinne.

Schließlich ist aus der US-PS 3 717 151 eine Vorrichtung zur Verwendung im Zusammenhang mit Kathetern, z. B. zur Wunddränage, bekannt, mit der die Aufgabe erfüllt werden soll, diese Vorrichtung so im Innern des Körpers eines Lebewesens anordnen zu können, daß die einmal positionierte Vorrichtung sich nicht zufällig lösen kann. Hierzu besteht die Vorrichtung im wesentlichen aus zwei koaxial angeordneten, relativ zueinander verschiebbaren Hülsen sowie aus dem distalen Ende der Außenhülse ausfahrbaren Greiffingern und einem auf der Außenhülse verschiebbaren Festlegekragen. Nach dem Einführen dieser Vorrichtung in eine Wunde oder in eine Körperhöhle, z. B. den Magen einer Kuh, werden die Greiffinger aus dem distalen Ende ausgefahren. Dabei spreizen sich die Greiffinger in radialer Richtung nach außen und legen sich, in diesem Beispiel, gegen die Magenwand. Anschließend wird der Festlegekragen auf der Außenhülse zum distalen Ende hin verschoben, bis er z. B. die Haut berührt, und wird dort festgelegt. Somit ist die gesamte Vorrichtung gegen zufälliges Herausfallen aus dem Körper und/oder Hineinfallen in die Körperhöhle gesichert. Ein Hinweis auf eine Anwendung zum Fixieren von Hohlorganen findet sich in dieser Schrift nicht.

Was das Vermessen von Hohlorganen betrifft, sei auf die DE-PS 33 30 921 verwiesen, die eine optiklose Sonde zur Bestimmung des Innenmaßes von Hohlorganen, insbesondere der Gebärmutterhöhlke, beschreibt. Bei diesem Instrument werden zwei am distalen Ende der Sonde vorgesehene, auseinanderspreizbare Fühler durch axiales Verschieben eines stabförmigen Übertragungsgliedes bis zur Berührung der Innenwand des Hohlorgans aufgespreizt. Am proximalen Ende dieser Sonde ist eine Anzeigevorrichtung, z. B. eine Meßuhr, angeordnet, auf der das Meßergebnis abgelesen werden kann. Auch diese Vorrichtung ist nicht in der Lage, das Ausweichen des zu behandelnden Hohlorganes während der Behandlung zu vermeiden.

Es ist daher die Aufgabe der Erfindung, ein Endoskop der einleitend angeführten Art so zu verbessern, daß es möglich ist, zur Betrachtung, Vermessung und Behandlung von Hohlorganen von Lebewesen solche Hohlorgane gegen eine äußere, festere Struktur ziehen und daran fixieren zu können.

Diese Aufgabe wird gelöst durch eine in dem Außenschaft führbare Haltevorrichtung mit einer Vielzahl von distal aus dem Außenschaft heraus-und hineinbewegbaren, elastisch verformbaren Hakenelementen, durch auf dem Außenschaft einstellbar und feststellbar angeordnete Anschlagmittel, durch eine im proximalen Bereich des Außenschaftes vorgesehene Verstelleinrichtung zum Verschieben der Haltevorrichtung in dem Außenschaft und durch eine Anzeigevorrichtung zur Kontrolle der Stellung der Verstelleinrichtung.

Mit diesem Endoskop laßt sich ein zu behandelndes Hohlorgan unter optischer Kontrolle wirksam festlegen. Weiter kann mit dem gleichen Endoskop z. B. der verbliebene Querschnitt eines pathologisch veränderten röhrenförmigen Hohlorgans dadurch bestimmt werden, daß der Spreizweg der Hakenelemente bis zur optisch kontrollierbaren Anlage an der Organwand ablesbar ist. Auch steht nun mehr Raum für die Benutzung der Behandlungsinstrumente zur Verfügung.

Bevorzugte Ausführungen des Erfindungsgegenstandes sind den Unteransprüchen zu entnehmen. Dabei sind besonders hervorzuheben: Die Ausbildung der Hakenelemente aus einem hochelastischen Draht oder Band, welche Ausführung eine hohe Zahl von Biegewechseln verträgt; das durch die Rückbiegung der distalen Enden der Hakenelemente erzielbare atraumatische Verhalten der Hakenelemente beim Kontakt mit der Organwand und schließlich die Meßskala im Bereich der Verstelleinrichtung, deren Teilung vorzugsweise proportional dem Spreizweg der Hakenelemente ist und damit einen direkten Aufschluß über die jeweilige Stellung derselben gibt.

Die Erfindung ist nachstehend anhand zweier, in der Zeichnung dargestellter Ausführungsbeispiele näher erläutert. Es zeigen:
Figur 1 ein erstes Endoskop in Seitenansicht,
Figur 2 das distale Ende des Endoskopes nach Figur 1 mit ausgeschobenen Hakenelementen, im Längsschnitt dargestellt,
Figur 3 eine Endansicht auf das distale Ende des Endoskopes mit ausgeschobenen Hakenelementen,
Figur 4 einen Axialschnitt durch das proximale Ende des Endoskopes,
Figur 5 einen Querschnitt durch das Endoskop nach der Linie V-V in Fig. 1,
Figur 6 das Endoskop im Einsatz, schematisch dargestellt,
Figur 7 ebenfalls in schematischer Darstellung die Anwendung des Endoskopes als Meßinstrument,
Figur 8 + 9 weitere Ausführungsformen des Endoskopes in teilweiser und axial geschnittener Darstellung,
Figur 10 eine Endansicht auf die Darstellung nach Fig. 9.

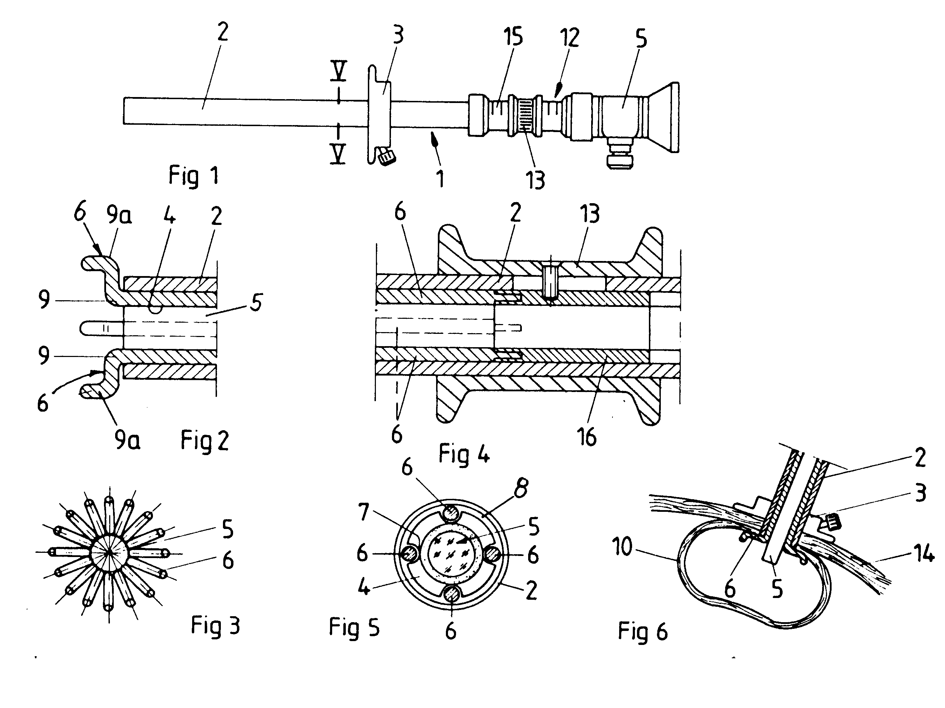

Nach den Figuren 1, 2 und 5 besteht das Endoskop 1 im wesentlichen aus einem Außenschaft 2 mit einer darauf verschiebbar angebrachten Anschlagscheibe 3, aus einem Innenschaft 4 sowie aus einer in den Innenschaft 4 einführbaren Optik 5.

In einem Ringraum 8 zwischen dem Außenschaft 2 und der Optik 5 ist gemäß den Figuren 2 und 5 eine Mehrzahl von Hakenelementen 6 angeordnet und axial geführt. Die Führung kann durch Längsnuten 7 in der Außenfläche des Innenschaftes 4 erfolgen oder durch einen mit entsprechenden Ausnehmungen versehenen Führungsring. Die Hakenelemente 6 bestehen aus einem längeren, hochelastischen Draht oder Band und weisen im Bereich ihres distalen Endes eine immanente Vorbiegung 9 auf, die sich beim Hinausschieben des Hakenelementes über das distale Ende des Außenschaftes 2 hinaus, wie in Figur 2 gezeigt, in radialer Richtung über den Außenschaft 2 hinaus spreizt und dabei als Hakenteil ausbildet. Um einen traumatischen Effekt sicher zu vermeiden, ist das vorgebogene Ende jedes Hakenelementes 6 an seinem distalen Ende mit einer weiteren, entgegen der genannten Vorbiegung verlaufenden, immanenten Rückbiegung 9a versehen. Die Länge der Hakenelemente 6 ist so gewählt, daß sie vollständig in den Außenschaft 2 des Instrumentes 1 hineingezogen werden können, wodurch vermieden wird, daß beim Einführen des Endoskopes 1 in ein Hohlorgan 10 oder 11 eines Lebewesens eine zusätzliche Traumatisierung durch die Hakenelemente 6 erfolgt. Die Hakenelemente 6 sind gemäß Fig. 4 mit ihrem proximalen Ende an einer in dem Ringraum 8 zwischen dem Außenschaft 2 und der Optik 5 verschiebbaren Hülse 16 festgelegt. Die Hülse 16 ist über eine Verbindungsschraube mit einer Stelleinrichtung 13 in Form einer Schiebehülse im Bereich der Handhabe 12 des Instrumentes 1 verbunden.

Die Wirkungsweise dieser Ausführungsform des Endoskopes ist folgende:
Zur Behandlung eines Hohlorgans 10, z. B. desjenigen nach Figur 6, wird zunächst das Endoskop 1 mit seinem Außenschaft 2 in dieses Hohlorgan eingeführt, wobei die Hakenelemente 6 in den Außenschaft 2 eingezogen sind. Nach dem Einführen wird die Schiebehülse 13 an der Handhabe 12 des Endoskopes 1 distalwärts vorgeschoben, was bewirkt, daß die immamenten Hakenteile 9 über das distale Ende des Außenschaftes 2 hinausgeschoben werden. Dadurch wird die äußere Abstützung der Hakenteile 9 fortschreitend aufgehoben, so daß sie sich zunehmend aufspreizen und als Haken ausbilden. Sie erreichen dadurch schließlich die Innenwand des Hohlorganes 10, an der sie sich anlegen. In dieser Stellung wird das Endoskop 1 mitsamt dem Hohlorgan 10 gegen eine festere Gewebestruktur, z. B. gegen die Bauchdecke 14, bis zur Anlage an dieser zurückgezogen und sodann die Anschlagscheibe 3 bis an die Außenseite dieser festeren Gewebestruktur herangeschoben und in der Anschlagstellung fixiert.

Somit ist das Hohlorgan 10 sicher fixiert und kann endoskopisch genau untersucht und behandelt werden.

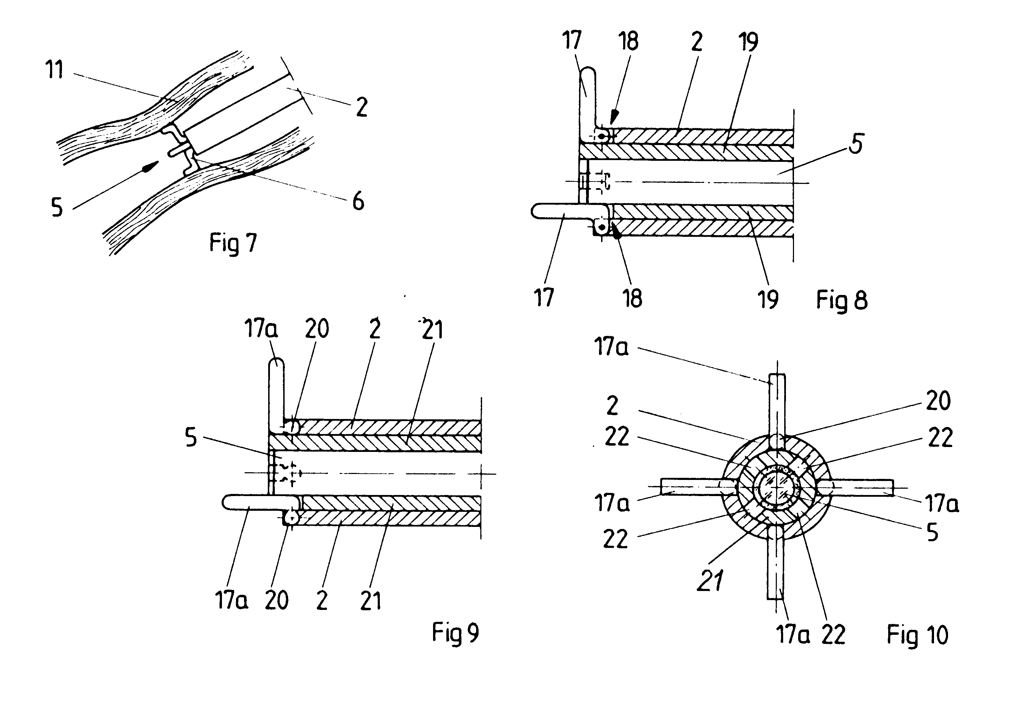

Um mit dem Endoskop 1 Messungen in Hohlorganen vornehmen zu können, ist im Bereich der proximalen Handhabe 12 eine Maßeinteilung 15 vorgesehen, auf der das der Entfernung der Enden von sich gegenüberliegenden Hakenteilen 9 entsprechende Maß abgelesen werden kann. Bei röhrenförmigen Hohlorganen 11 (Figur 7) wird das Endoskop 1 mit Hilfe der Kontrolle durch die Optik 5 an den interessierenden Bereich herangebracht und sodann die immanenten Hakenteile 9 aus dem distalen Ende des Außenschaftes 2 herausgeschoben, bis sie die Wand des Hohlorgans 11 berühren. Hierdurch kann z. B. bei pathologischen Veränderungen der noch verbliebene freie Durchgang eines röhrenförmigen Hohlorgans gemessen werden.

In abgeänderter Ausführungsform nach Figur 8 kann das vorgeschlagene Endoskop hinsichtlich der Hakenelemente auch so ausgebildet sein, daß eine Vielzahl von starren, L-förmigen Hakenelementen 17 in axialen Schlitzen 18 am distalen Ende des Außenschaftes 2 umfangsmäßig verteilt und radial verschwenkbar angelenkt ist, wie es Figur 8 zeigt. Die Anlenkung und die Formgestaltung der Hakenelemente 16 ist dabei so gewählt, daß die Hakenelemente in ihrer Nichtfunktionsstellung in bezug auf den Außenschaft 2 mit Hilfe der Optik 5 nicht radial nach außen vorstehen, wie es in der unteren Hälfte in Figur 8 gezeigt ist. Die obere Hälfte in Figur 8 zeigt die Funktionsstellung der Hakenelemente 17. Die Schwenkbetätigung der Hakenelemente erfolgt durch axiale Vorbewegung des Innenschaftes 19 in dem Außenschaft 2, wobei der auch die Optik 5 aufnehmende Innenschaft zunächst gegen das Knie der L-förmigen Hakenelemente drückt, um diese Elemente radial auszuschwenken, wie aus Figur 7 unten erkennbar ist, und dann die anderen Schenkel der Hakenelemente durch seine vorgeschobene Stellung radial nach außen geschwenkt hält, wie es in Figur 8 oben gezeigt ist.

Bei dem noch weiteren Ausführungsbeispiel nach den Figuren 9 und 10 weist der Innenschaft 21 eine der Anzahl der im Außenschaft 2 mit Kugelgelenken 20 gelagerten Hakenelemente 17a entsprechende Anzahl von Ausnehmungen 22 auf, die dazu dienen, die Hakenelemente 17a in achsparalleler Stellung, wie im unteren Teil der Figur 9 dargestellt, vermittels der Optik 5 zu fixieren.

Zum Ausschwenken der Hakenelemente 17a, z. B. in die Stellung, wie im oberen Teil der Figur 9 dargestellt, werden zunächst die Optik 5 und der Innenschaft 21 soweit proximalwärts zurückgezogen, bis das distale Ende des Innenschaftes 21 vollständig proximalseits des proximalen Endes der Haken 17a liegt. Anschließend wird der Innenschaft 21 so gedreht, daß die Aussparungen 22, wie in Figur 10 dargestellt, nicht mehr in Richtung der Hakenelemente 17a zeigen. Durch Vorschieben des Innenschaftes 21 in Richtung auf die Hakenelemente 17a werden diese durch den Schaft 21 in radialer Richtung ausgeschwenkt.

Zum Rückführen der Hakenelemente 17a in die achsparallele Ruhestellung wird der Innenschaft wieder gedreht, bis sich die Positionen der im Außenschaft 2 angelenkten Hakenelemente 17a und die Ausnehmungen 22 des Innenschaftes 21 decken, so daß die Hakenelemente, z. B. beim Herausziehen des Instrumentes aus der Körperhöhle, in die Ruhestellung zurückfallen.

Um beim Einführen des Endoskopes in eine Körperhöhle keine unnötigen Traumatisierungen herbeizuführen, werden die Hakenelemente 17a auch in dieser Ausführungsform in ihrer Ruhelage einfach durch distalwärtiges Vorschieben der Optik 5 fixiert.

Das erfindungsgemäße Endoskop 1 kann auch als flexibles Instrument mit flexiblen Schäften 2, 4 bzw. 19, 21 anstelle starrer Schäfte und mit einer flexiblen Optik anstelle einer starren Optik 5 ausgebildet sein.

## Patentansprüche

1. Endoskop zum Einführen in ein Hohlorgan eines Lebewesens, umfassend einen hohlen Außenschaft (2) und eine in diesem angeordnete Optik (5), gekennzeichnet durch eine in dem Außenschaft (2) geführte Haltevorrichtung (4) mit einer Vielzahl von distal aus dem Schaft heraus- und hineinbewegbaren, elastisch verformbaren Hakenelementen (6, 17), durch auf dem Außenschaft (2) einstellbar und feststellbar angeordnete Anschlagmittel (3), durch eine im proximalen Bereich des Außenschaftes (2) vorgesehene Verstelleinrichtung (13) zum Verschieben der Haltevorrichtung in dem Außenschaft (2) und durch eine Anzeigevorrichtung (15) zur Kontrolle der Stellung der Verstelleinrichtung.

2. Endoskop nach Anspruch 1, dadurch gekennzeichnet, daß die Hakenelemente (6) je aus einem längeren, hochelastischen Draht mit einem distalen, elastisch verformbaren und selbsttätig spreizbaren Hakenteil (9) bestehen.

3. Endoskop nach Anspruch 2, dadurch gekennzeichnet, daß das durch Biegen hergestellte Hakenteil (9) jedes Hakenelementes (6) an seinem distalen Ende eine weitere, entgegen der ersten Vorbiegung verlaufende, elastisch verformbare Rückbiegung (9a) aufweist.

4. Endoskop nach einem der Ansprüche 1 bis 3, dadurch gekennzeichnet, daß ein die Optik (5) aufnehmender Innenschaft (4) in einem Ringraum (8) zwischen der Optik und dem Außenschaft (2) vorgesehen ist und daß die Hakenelemente (6) in Ausnehmungen (7) des Innenschaftes untergebracht sind.

5. Endoskop nach Anspruch 4, dadurch gekennzeichnet, daß die Hakenelemente (6) mit ihrem proximalen Ende an einer in dem Ringraum (8) zwischen dem Außenschaft (2) und der Optik (5) verschiebbaren Hülse (16) festgelegt sind und daß die Verstelleinrichtung (13) für die Hakenelemente (6) aus einer proximalseitig auf dem Außenschaft (2) geführten Schiebehülse besteht, die mit der die Hakenelemente (6) tragenden Hülse (16) durch ein starres Verbundungsglied verbunden ist.

6. Endoskop zum Einführen in ein Hohlorgan eines Lebewesens, umfassend einen hohlen Außenschaft (2) und eine in diesem angeordnete Optik (5), dadurch gekennzeichnet, daß eine Vielzahl starrer, L-förmiger Hakenelemente (17, 17a) am distalen Ende des Außenschaftes (2) radial verschwenkbar angelenkt ist, daß ein an dem Außenschaft geführter, die Optik (5) aufnehmender, axial bewegbarer Innenschaft (19, 21) für die Auschwenkbetätigung der Hakenelemente vorgesehen ist, daß auf dem Außenschaft (2) Anschlagmittel (3) einstellbar und feststellbar angeordnet sind, daß eine im proximalen Bereich des Außenschaftes (2) vorgesehene Verstelleinrichtung (13) zum Verschieben des Innenschaftes (19, 21) vorgesehen ist und daß eine Anzeigevorrichtung (15) zur Kontrolle der Stellung der Verstelleinrichtung vorgesehen ist.

7. Endoskop nach Anspruch 1 oder 6, dadurch gekennzeichnet, daß die Anzeigevorrichtung (15) aus einer auf den Außenschaft (2) vorgesehenen Meßskala besteht.
